# EUROPEAN PATENT SPECIFICATION

(11) **EP 4 163 632 B1**
(45) Date of publication and mention of the grant of the patent: **23.09.2026**
(21) Application number: 21865843.3
(22) Date of filing: 20.08.2021
(51) Int. Cl.: G01N 33/543, G01N 33/53, B01L 3/00

(54) **MICROFLUIDIC IMMUNODETECTION CHIP AND MICROFLUIDIC LINEAR IMMUNODETECTION METHOD**
MIKROFLUIDISCHER IMMUNDETEKTIONSCHIP UND MIKROFLUIDISCHES LINEARES IMMUNDETEKTIONSVERFAHREN
PUCE D'IMMUNODÉTECTION MICROFLUIDIQUE ET MÉTHODE D'IMMUNODÉTECTION LINÉAIRE MICROFLUIDIQUE

(30) Priority: 08.09.2020 CN 202010934760
(43) Date of publication of application: 12.04.2023
(73) Proprietor: Shenzhen Yhlo Biotech Co., Ltd., 518116 Shenzhen (CN)
(72) Inventor: ZHENG, Yanping, Shenzhen, Guangdong 518116 (CN); CHENG, Xiaoyu, Shenzhen, Guangdong 518116 (CN); WEI, Daoshun, Shenzhen, Guangdong 518116 (CN); QIAN, Chungen, Shenzhen, Guangdong 518116 (CN); HU, Kunhui, Shenzhen, Guangdong 518116 (CN)
(74) Representative: Michalski Hüttermann & Partner mbB
(86) International application number: PCT/CN2021/113725
(87) International publication number: WO 2022/052787

(56) References cited:
- CN-A- 104 502 594
- CN-A- 105 562 130
- CN-A- 109 212 201
- CN-A- 109 884 328
- CN-A- 111 965 345
- CN-U- 210 787 395
- US-A1- 2011 020 194
- CHUN-CHE LIN ET AL: "Microfluidic Immunoassays", JOURNAL OF THE ASSOCIATION FOR LABORATORY AUTOMATION, vol. 15, no. 3, 1 June 2010 (2010-06-01), pages 253 - 274, XP055275760, ISSN: 1535-5535, DOI: 10.1016/j.jala.2010.01.013
- JIWOON PARK: "Lab-on-a-Disc for Fully Integrated Multiplex Immunoassays", ANALYTICAL CHEMISTRY, vol. 84, no. 5, 13 February 2012 (2012-02-13), US, pages 2133 - 2140, XP093154204, ISSN: 0003-2700, DOI: 10.1021/ac203163u
- O. STROHMEIER ET AL: "Centrifugal microfluidic platforms: advanced unit operations and applications", CHEMICAL SOCIETY REVIEWS, vol. 44, no. 17, 1 January 2015 (2015-01-01), UK, pages 6187 - 6229, XP055423852, ISSN: 0306-0012, DOI: 10.1039/C4CS00371C
- MINGHUI TANG ET AL: "A Review of Biomedical Centrifugal Microfluidic Platforms", MICROMACHINES, vol. 7, no. 2, 6 February 2016 (2016-02-06), pages 26, XP055319405, DOI: 10.3390/mi7020026

## Description

This application claims priority to Chinese Patent Application No. 202010934760.5, filed on September 08, 2020 with Chinese patent office, entitled "MICROFLUIDIC IMMUNOASSAY CHIP AND MICROFLUIDIC LINE IMMUNOAS SAY METHOD".

### TECHNICAL FIELD

The present disclosure relates to the technical field of microfluidics, and in particular to a microfluidic immunoassay chip and a microfluidic line immunoassay method.

### BACKGROUND

Microfluidics, as an emerging science and technology, has been applied to many fields such as chemistry, biology, engineering, physics, etc. Microfluidics is highly interdisciplinary, brings breakthroughs in aspects of temporality, spatiality, and precise manipulation of analysis objects, and can solve many key problems in life analysis. Microfluidics can integrate the detections and experiments that otherwise can only be conducted in laboratories onto a small chip, which not only saves the costs of consumables and time, but more importantly, can integrate multiple detection technologies into a whole, thereby increasing the detection efficiency.

A microfluidic immunochip assay technique is a new micro total analysis and detection method developed in recent years. It combines microfluidics with an immunoassay, building an immunoassay platform on a microfluidic chip, and thus can take advantages of the two assay methods. A very small amount of reagents is needed in the microfluidic assay, which greatly decreases the consumption of expensive immune reagents such as antibodies. Due to the precise manipulation of fluids at micro and nano scales and the highly integrated analytical process, not only the speed of the antigen-antibody reaction is increased and the reaction time is effectively shortened, but also the operating process of the immunoassay is significantly simplified. The unique biological recognition mechanism of the immunoassay further enhances the specificity of the microfluidic assay and renders characteristics such as high sensitivity, high precision, high specificity, etc. to the microfluidic immunoassay chip. Thus, the detection of allergic diseases and autoimmune diseases can be further improved and become more automated and miniaturized by combining the immunoassay with the microfluidic assay.

However, the inner surface of the conventional chip is difficult to be modified and thus difficult to stably and efficiently fix various capture antigens or capture antibodies, which hinders a further wide application of the chip. Related technologies are known from CN109884328A, CN104502594A and CN105562130A. CN109884328A discloses a lateral flow immunodetection system based on a centrifugal microfluidic system.

### SUMMARY

A microfluidic immunoassay chip as defined in claim 1 and a microfluidic line immunoassay method as defined in claim 14 are provided according to various embodiments. Further aspects and preferred embodiments are defined in the dependent claims. Aspects, embodiments, examples, and implementations of the present disclosure that do not fall within the scope of the appended claims do not form part of the invention and are merely provided for illustrative purposes.

A microfluidic immunoassay chip of the invention is defined in accordance with claim 1.

A microfluidic line immunoassay method of the invention is defined in accordance with claim 14.

In order to understand the technical means of the present disclosure more clearly and to implement the present disclosure according to the content of the specification, hereafter the preferred embodiments of the present disclosure will be detailed described as follows in conjunction with the accompanying drawings.

### BRIEF DESCRIPTION OF THE DRAWINGS

In order to illustrate the solutions in the embodiments of the present disclosure or in the conventional technology more clearly, the drawings used in the description of the embodiments or the conventional technology will be described briefly as follows. Apparently, the following described drawings are merely some embodiments of the present disclosure, and other drawings can be derived from these drawings by one of ordinary skill in the art without any creative effort.
FIG. 1 is a front view of a microfluidic immunoassay chip according to an embodiment.
FIG. 2 is a partially enlarged schematic view of a detection unit as shown in FIG. 1.
FIG. 3 is a partially enlarged schematic view of the detection unit as shown in FIG. 2.
FIG. 4 is a flowchart of a microfluidic line immunoassay method according to an embodiment.

### DETAILED DESCRIPTION

In order to facilitate understanding of the present disclosure, the present disclosure will be comprehensively described and the preferred embodiments of the present disclosure will be provided hereafter. However, the present disclosure can be implemented in many different forms and therefore is only limited by the terms of the claims. In contrast, the purpose of providing these embodiments is to thoroughly and comprehensively understand the present disclosure.

Unless otherwise defined, all the technical and scientific terms used herein have the same meaning as commonly understood by the person skilled in the art to which the present disclosure belongs. Herein, the terms used in the description of the present disclosure are merely for the purpose of describing specific examples without limiting the present disclosure. The term "and/or" used herein includes any and all combinations of one or more of the associated listed items.

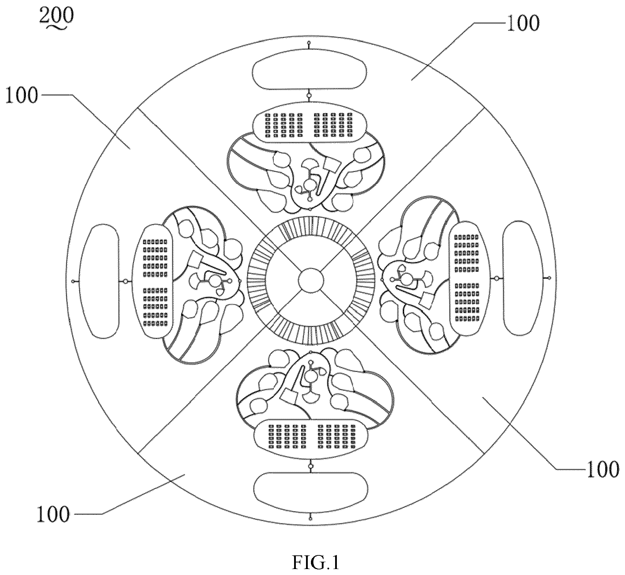

As shown in FIG. 1, a microfluidic immunoassay chip 200 according to an embodiment of the present disclosure includes a plurality of detection units 100. The microfluidic immunoassay chip 200 is substantially circular in shape and has a rotation center O defined in its central portion. The rotation center O is the center of rotation of the microfluidic immunoassay chip 200 in centrifugal operation.

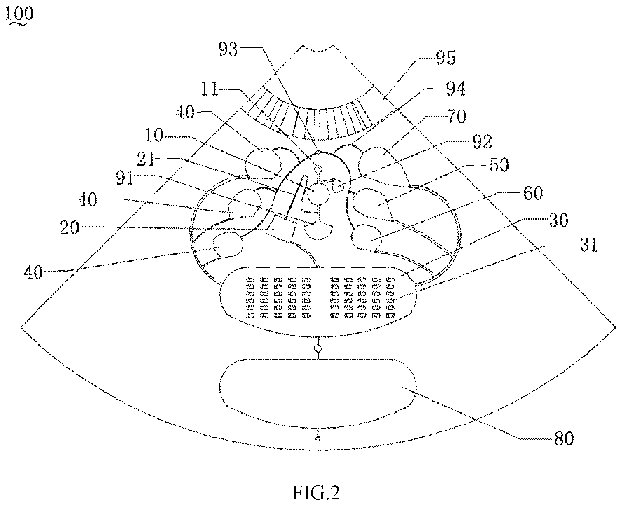

As shown in FIG. 2, each detection unit 100 includes a loading cell 10, a reaction and detection cell 30, a washing cell 40, an enzyme storing cell 50, a substrate cell 60, a termination cell 70, and a waste liquid cell 80.

The loading cell 10 is provided with a loading hole 11 through which a sample is to be added. The reaction and detection cell 30 is in communication with the loading cell 10. A detection membrane strip 31 is disposed in the reaction and detection cell 30 and coated with a capture antigen or a capture antibody. The washing cell 40 is configured to be pre-filled with a buffer solution and is in communication with the reaction and detection cell 30. The enzyme storing cell 50 is configured to be pre-filled with an enzyme labeled antibody and is in communication with the reaction and detection cell 30. The substrate cell 60 is configured to be pre-filled with a reaction substrate for the above enzyme labeled antibody, and the substrate cell 60 is in communication with the reaction and detection cell 30. The termination cell 70 is configured to be pre-filled with a reaction terminating liquid and is in communication with the reaction and detection cell 30. The waste liquid cell 80 is configured to receive a waste liquid and is in communication with the reaction and detection cell 30.

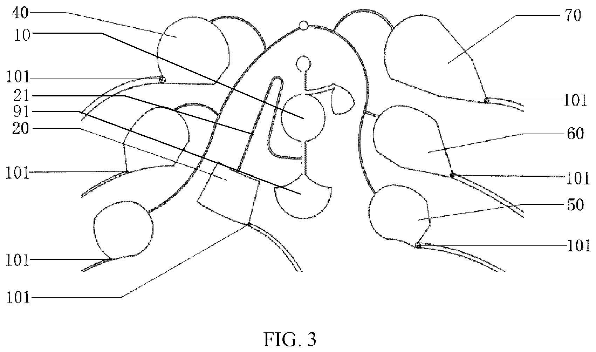

The reaction and detection cell 30 is further away from the rotation center O than the loading cell 10, the washing cell 40, the enzyme storing cell 50, the substrate cell 60, the termination cell 70, and the waste liquid cell 80. As shown in FIG. 3, a valve 101 is disposed between the reaction and detection cell 30 and each of the loading cell 10, the washing cell 40, the enzyme storing cell 50, the substrate cell 60, the termination cell 70, and the waste liquid cell 80.

In use of the above-described microfluidic immunoassay chip 200, a sample liquid can be loaded into the loading cell 10 through the loading hole 11. Then the microfluidic immunoassay chip 200 is centrifuged to force the sample liquid into the reaction and detection cell 30. The direction and the amplitude of the centrifugation are constantly changed to produce a back and forth mixing and vibration effect on the liquid. In the meanwhile, the capture antigen or the capture antibody on the detection membrane strip 31 captures a target substance in the sample liquid. Thereafter, the liquid is discharged into the waste liquid cell 80 under the action of centrifugation. The buffer solution in the washing cell 40 is released into the reaction and detection cell 30, vibrated back and forth to perform a first washing, and then discharged into the waste liquid cell 80. The enzyme labeled antibody in the enzyme storing cell 50 is released into the reaction and detection cell 30, mixed and incubated, and then discharged into the waste liquid cell 80. The buffer solution in the washing cell 40 is released again to perform a second washing and then discharged into the waste liquid cell 80. The reaction substrate in the substrate cell 60 is released into the reaction and detection cell 30 to achieve a chromogenic reaction and then discharged into the waste liquid cell 80. The buffer solution in the washing cell 40 is released again into the reaction and detection cell 30 to perform a third washing and then discharged into the waste liquid cell 80. Then the reaction terminating liquid in the termination cell 70 is released into the reaction and detection cell 30 to terminate the reaction. After the reaction terminating liquid is evacuated, the reaction result is acquired from the reaction and detection cell 30 and analyzed. The microfluidic immunoassay chip 200 combines the line immunoblotting with the microfluidic assay, utilizes the detection membrane strip 31 disposed in the reaction and detection cell 30 and coated with the capture antigen or the capture antibody as a solid phase detection platform. The microfluidic immunoassay chip 200 requires no modification on the inner surface of the chip, and can stably and efficiently fix various capture antigens or capture antibodies. In addition, the microfluidic immunoassay chip 200 takes advantages of integration and miniaturization of the chip, addresses the shortages of the line immunoblotting using membrane strips such as the complicated and time consumed operation, not only saves the reagents and time, but also requires no specialized personnel to operate, which decreases the detection cost.

It is to be understood that the above-described capture antigen or capture antibody can bind the target substance to be detected in the sample liquid, so that the target substance to be detected is fixed onto the detection membrane strip 31. The enzyme labeled antibody is then bound to the target substance to be detected in the sample liquid, so as to be fixed onto the detection membrane strip 31 and react with the subsequent reaction substrate to develop color change.

In the present embodiment, the detection membrane strip 31 can be made from a membrane strip commonly used in line immunoblotting. The line immunoblotting refers to an assay that transfers antigens or antibodies onto a membrane such as a nitrocellulose membrane as separate bands through electrophoresis due to the differences in molecular mass and structure, which can be used to detect a corresponding antibody in combination with an enzyme labeled antibody and a chromogenic substrate.

In some embodiments, the microfluidic immunoassay chip 200 includes four detection units 100 evenly distributed around the rotation center O to detect four samples simultaneously, which is suitable for various detection samples such as whole blood, plasma, saliva, etc. Of course, in other embodiments, the microfluidic immunoassay chip 200 can be in another shape such as rectangle, polygon, etc. The number of the detection units 100 can be one, two, three, five, seven, etc.

As shown in FIG. 2, the reaction and detection cell 30 is provided with a plurality of detection slots spaced from each other. A plurality of detection membrane strips 31 are correspondingly embedded in the plurality of detection slots. Each of the detection membrane strips 31 is coated with one or more types of capture antigens or capture antibodies thereon. In this way, multiple target substances can be detected at one time, thereby increasing the detection efficiency.

In the present embodiment, the plurality of detection slots are arranged in a rectangular array. In the embodiment as shown in FIG. 1, there are 60 detection slots arranged in 6 rows and 10 columns. It is to be understood that the arrangement manner is not limited thereto and can be adjusted according to needs. In the present embodiment, the detection membrane strips 31 are rectangular membrane strips and the detection slots are rectangular slots. Of course, in other embodiments, the detection membrane strips 31 and the detection slots can be in other shapes such as triangle, circle, etc.

In a specific embodiment, a height difference between a top surface of the detection membrane strip 31 and a top edge of a side wall of the detection slot is 20 µm to 200 µm but is not limited thereto. In this way, the target substance in the sample liquid can be in sufficient contact with the detection membrane strip 31 to have a reaction, which increases the detection efficiency and sensitivity. In the present embodiment, a bottom surface of the detection membrane strip 31 can directly abut against a bottom wall of the detection slot, or there can be a gap between the bottom surface of the detection membrane strip 31 and the bottom wall of the detection slot, so that the target substance in the sample liquid can be in more sufficient contact with the detection membrane strip 31 to have the reaction.

In a specific embodiment, the detection unit 100 further includes a dilution cell 20 configured to be pre-filled with a diluent for diluting the sample. The dilution cell 20 is further away from the rotation center O than the loading cell 10 and is closer to the rotation center O than the reaction and detection cell 30. The dilution cell 20 is in communication with the loading cell 10 through a siphonic channel 21. The reaction and detection cell 30 is in communication with the loading cell 10 through the dilution cell 20. In addition, a valve 101 is further disposed between the reaction and detection cell 30 and the dilution cell 20 to control the release of the liquid from the dilution cell 20. Optionally, the valves 101 are paraffin valves, photosensitive wax valves, or press valves, but are not limited thereto.

As shown in FIG. 3, the siphonic channel 21 from the end thereof being in communication with the loading cell 10 extends in a direction towards the rotation center O, and then bends and extends in a direction away from the rotation center O, thereby being in communication with the dilution cell 20. The bending location is closer to the rotation center O than the loading cell 10. In this way, in centrifugation at a high speed, the siphonic channel 21 is unable to be actuated because the centrifugal force is larger than the capillary force. When the centrifugal force is decreased into a suitable range such that the capillary force is larger than the centrifugal force, the siphonic channel 21 is actuated. At this time, the centrifugal rotation speed can be increased to maintain the siphonic effect, so that a fixed amount of liquid can be forced into the dilution cell 20 due to the siphonic effect, diluted and mixed sufficiently, which is advantageous for improving the detection effect.

As shown in FIG. 3, the detection unit 100 further includes a precipitation cell 91 being in communication with the loading cell 10. The precipitation cell 91 is further away from the rotation center O than the loading cell 10. In this way, after the sample liquid is loaded into the loading cell 10, the sample liquid is stratified by centrifugation and an impurity in the sample liquid is precipitated into the precipitation cell 91 and is thus prevented from subsequently flowing into the reaction and detection cell 30, which is advantageous for increasing the detection efficiency and sensitivity. In an embodiment, the loading cell 10, the precipitation cell 91, the reaction and detection cell 30, and the waste liquid cell 80 are sequentially arranged in a radical direction away from the rotation center O.

In a specific embodiment, the capture antigen is an autoantibody target detection substance or an allergen. Optionally, the autoantibody target detection substance is selected from dsDNA, nucleosome, Ku antigen, histone, ssDNA, SM antigen, ribonucleoprotein (nRNP), ribosomal p protein (RIB-P), anti-mitochondrial antibody M2 (AMA-M2), Jo-1 antigen, desiccation syndrome antigen A (SSA), or desiccation syndrome antigen B (SSB); the allergen is selected from pollen, insect mite, mold, cat hair, dog epithelium, cockroach, house dust, or grass allergens, which are suitable for the inhaled allergen detection and the corresponding autoantibody detection. It is to be understood that the capture antigen or capture antibody coated onto the detection membrane strip 31 is not limited thereto and can be adjusted according to the target substance of the detection.

In a specific embodiment, there are at least three washing cells 40 each being in communication with the reaction and detection cell 30. In this way, the washing with the butter solution can be conveniently performed after each incubation step in the reaction and detection cell 30, which is advantageous for increasing the detection efficiency and sensitivity.

As shown in FIG. 3, the detection unit 100 further includes an excess sample cell 92 being in communication with the loading cell 10. A distance between the excess sample cell 92 and the rotation center O is equal to a distance between the loading cell 10 and the rotation center O, so that the excess sample cell 92 can be used to receive the excess sample liquid. In this way, when the sample liquid is loaded into the loading cell 10 for the centrifugation, the excess sample liquid can flow into the lateral excess sample cell 92 since the excess sample cell 92 and the loading cell 10 are located on the same latitude.

In a specific embodiment, the detection unit 100 further includes a venting hole 93 and a gas channel 94. The washing cell 40, the enzyme storing cell 50, the substrate cell 60, and the termination cell 70 are all in communication with the venting hole 93 through the gas channel 94. The venting hole 93 is closer to the rotation center O than the washing cell 40, the enzyme storing cell 50, the substrate cell 60, and the termination cell 70. The gas channel 94 can have a width of 50 µm to 100 µm and a height of 50 µm to 100 µm, which can balance the gas pressure inside the chip. In an embodiment, the venting hole 93 and the loading hole 11 each are sealed with a hydrophobic and gas-permeable film to prevent the generation of aerosol.

In a specific embodiment, each detection unit 100 is provided with a bar code 95 thereon to distinguish different samples in the detection.

In some embodiments, the processing methods of the microfluidic immunoassay chip 200 include CNC, laser engraving, soft lithography, 3D printing, injection molding, and other methods, but are not limited thereto.

In a specific embodiment, the microfluidic immunoassay chip 200 incudes a bottom plate and a top plate. The loading cell 10, the dilution cell 20, the reaction and detection cell 30, the washing cell 40, the enzyme storing cell 50, the substrate cell 60, and the termination cell 70, the waste liquid cell 80, the precipitation cell 91, and the excess sample cell 92 as described above are all defined in the bottom plate. The loading hole 11, the venting hole 93, and the gas channel 94 are all defined in the top plate. Optionally, the two chambers that are to be in communication with each other or the chamber and the hole that are to be in communication with each other are communicated through a microchannel. For example, the communication between the loading cell 10 and the precipitation cell 91, the communication between the dilution cell 20 and the reaction and detection cell 30, and the communication between the washing cell 40, the enzyme storing cell 50, the substrate cell 60, as well as the termination cell 70 and the venting hole 93, are all achieved by the microchannels.

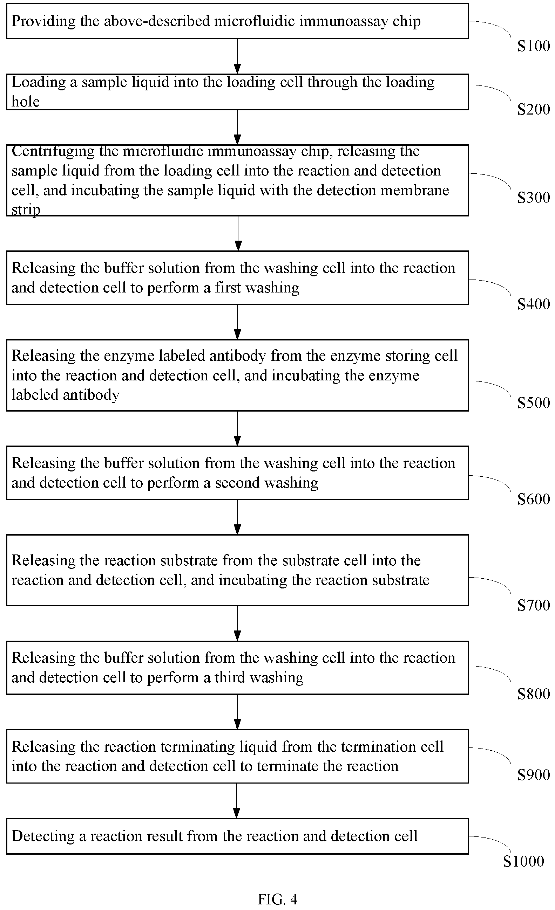

As shown in FIG. 4, a microfluidic line immunoassay method is further disclosed in an embodiment of the present disclosure, including the following steps:
S100, providing the above-described microfluidic immunoassay chip 200;
S200, loading a sample liquid into the loading cell 10 through the loading hole 11;
S300, centrifuging the microfluidic immunoassay chip 200 and opening the valve 101 between the loading cell 10 and the reaction and detection cell 30, so that the sample liquid is released into the reaction and detection cell 30, incubated with the detection membrane strip 31, and then discharged into the waste liquid cell 80;
S400, opening the valve 101 between the washing cell 40 and the reaction and detection cell 30, so that the buffer solution in the washing cell 40 is released into the reaction and detection cell 30 to perform a first washing and then discharged into the waste liquid cell 80;
S500, opening the valve 101 between the enzyme storing cell 50 and the reaction and detection cell 30, so that the enzyme labeled antibody in the enzyme storing cell 50 is released into the reaction and detection cell 30, incubated, and then discharged into the waste liquid cell 80;
S600, opening the valve 101 between the washing cell 40 and the reaction and detection cell 30, so that the buffer solution in the washing cell 40 is released into the reaction and detection cell 30 to perform a second washing and then discharged into the waste liquid cell 80;
S700, opening the valve 101 between the substrate cell 60 and the reaction and detection cell 30, so that the reaction substrate in the substrate cell 60 is released into the reaction and detection cell 30, incubated, and then discharged into the waste liquid cell 80;
S800, opening the valve 101 between the washing cell 40 and the reaction and detection cell 30, so that the buffer solution in the washing cell 40 is released into the reaction and detection cell 30 to perform a third washing and then discharged into the waste liquid cell 80;
S900, opening the valve 101 between the termination cell 70 and the reaction and detection cell 30, so that the reaction terminating liquid is released into the reaction and detection cell 30 to terminate the reaction and then discharged into the waste liquid cell 80;
S1000, detecting and analyzing the reaction result in the reaction and detection cell 30.

In a specific embodiment, a sample of whole blood is loaded into the loading cell 10 through the loading hole 11. Then the centrifugation is started to separate the plasma components from the whole blood. Then the centrifugal speed is decreased until the siphonic channel 21 is actuated, and then the centrifugal speed is increased and maintained, so that a fixed amount of plasma can be forced into the dilution cell 20 due to the siphonic effect. After the sample enters the dilution cell 20, the centrifugal direction is constantly changed so that the sample is subjected to a vibration and mixing effect. When the sample is mixed uniformly, the valve 101 is opened, allowing the sample solution to enter the reaction and detection cell 30 and to be incubated with the detection membrane strip 30.

The sample liquid after the incubation is discharged into the waste liquid cell 80 under the action of centrifugation at a high speed, before the valve 101 between the washing cell 40 and the reaction and detection cell 30 is opened. As such, the buffer solution in the washing cell 40 is released into the reaction and detection cell 30 to perform the first washing and then discharged into the waste liquid cell 80. The valve 101 between the enzyme storing cell 50 and the reaction and detection cell 30 is opened, so that the enzyme labeled antibody in the enzyme storing cell 50 is released into the reaction and detection cell 30, incubated, and then discharged into the waste liquid cell 80. The valve 101 between the washing cell 40 and the reaction and detection cell 30 is opened, so that the buffer solution in the washing cell 40 is released into the reaction and detection cell 30 to perform a second washing and then discharged into the waste liquid cell 80. The valve 101 between the substrate cell 60 and the reaction and detection cell 30 is opened, so that the reaction substrate in the substrate cell 60 is released into the reaction and detection cell 30, incubated, and then discharged into the waste liquid cell 80. The valve 101 between the washing cell 40 and the reaction and detection cell 30 is opened, so that the buffer solution in the washing cell 40 is released into the reaction and detection cell 30 to perform a third washing and then discharged into the waste liquid cell 80. The valve 101 between the termination cell 70 and the reaction and detection cell 30 is opened, so that the reaction terminating liquid is released into the reaction and detection cell 30 to terminate the reaction and then discharged into the waste liquid cell 80. The reaction result is acquired from the reaction and detection cell 30 and analyzed.

The microfluidic line immunoassay method which adopts the above-described microfluidic immunoassay chip 200 not only can be used to assist the early screening and diagnosis of allergic diseases and autoimmune diseases, but also can be used in the detection of samples from various sources such as the environment and food. In addition, the types of molecules that can be detected are not limited to disease-related molecules. In fact, any antigens and antibodies can be detected by this method. Thus, the application of the method is universal.

The microfluidic line immunoassay method combines the line immunoblotting with the microfluidic assay technology, utilizes the detection membrane strip 31 disposed in the reaction and detection cell 30 and coated with the capture antigen or the capture antibody as a solid phase detection platform. The microfluidic immunoassay chip 200 requires no modification on the inner surface of the chip, and can stably and efficiently fix various capture antigens or antibodies. In addition, the microfluidic immunoassay chip 200 takes advantages of integration and miniaturization of the chip, addresses the shortages of the line immunoblotting using membrane strips such as the complicated and time consumed operation, not only saves the reagents and time, but also requires no specialized personnel to operate, which decreases the detection cost.

The technical features of the above-described embodiments can be combined arbitrarily. To simplify the description, not all possible combinations of the technical features in the above embodiments are described.

## Claims

1. A microfluidic immunoassay chip, comprising at least one detection unit (100) and having a rotation center (O) about which the microfluidic immunoassay chip is configured to provide a centrifugal operation, wherein the detection unit (100) comprises:
a loading cell (10) provided with a loading hole (11);
a reaction and detection cell (30) being in communication with the loading cell (10);
a washing cell (40) configured to be pre-filled with a buffer solution, the washing cell (40) being in communication with the reaction and detection cell (30);
an enzyme storing cell (50) configured to be pre-filled with an enzyme labelled antibody, the enzyme storing cell (50) being in communication with the reaction and detection cell (30);
a substrate cell (60) configured to be pre-filled with a reaction substrate for the enzyme labelled antibody, the substrate cell (60) being in communication with the reaction and detection cell (30);
a termination cell (70) configured to be pre-filled with a reaction terminating liquid, the termination cell (70) being in communication with the reaction and detection cell (30); and
a waste liquid cell (80) configured to receive a waste liquid, the waste liquid cell (80) being in communication with the reaction and detection cell (30);
wherein the reaction and detection cell (30) is further away from the rotation center (O) than the loading cell (10), the washing cell (40), the enzyme storing cell (50), the substrate cell (60), and the termination cell (70), and is closer to the rotation center (O) than the waste liquid cell (80);
**characterized in that** the reaction and detection cell (30) is provided with a plurality of detection membrane strips (31) coated with a capture antigen or a capture antibody, the reaction and detection cell (30) comprises a plurality of detection slots spaced from each other, and a detection membrane strip (31) is correspondingly embedded in each of the plurality of detection slots.

2. The microfluidic immunoassay chip of claim 1, further comprising a valve (101) disposed between the reaction and detection cell (30) and each of the loading cell (10), the washing cell (40), the enzyme storing cell (50), the substrate cell (60), and the termination cell (70).

3. The microfluidic immunoassay chip of claim 1, wherein a height difference between a top surface of the detection membrane strip (31) and a top edge of a side wall of the each of the plurality of detection slots is 20 µm to 200 µm.

4. The microfluidic immunoassay chip of claim 1, wherein the detection unit (100) further comprises a dilution cell configured to be pre-filled with a diluent; the dilution cell is further away from the rotation center (O) than the loading cell (10) and closer to the rotation center (O) than the reaction and detection cell (30); the dilution cell is in communication with the loading cell (10) through a siphonic channel (21); and the reaction and detection cell (30) is in communication with the loading cell (10) through the dilution cell.

5. The microfluidic immunoassay chip of claim 4, wherein the siphonic channel (21) from one end thereof being in communication with the loading cell (10) extends in a direction towards the rotation center (O), bends at a position closer to the rotation center (O) than the loading cell (10), and extends in a direction away from the rotation center (O), thereby being in communication with the dilution cell.

6. The microfluidic immunoassay chip of claim 1, wherein the detection unit (100) further comprises a precipitation cell (91) being in communication with the loading cell (10), and the precipitation cell (91) is further away from the rotation center (O) than the loading cell (10).

7. The microfluidic immunoassay chip of claim 1, wherein the capture antigen is an autoantibody target detection substance or an allergen.

8. The microfluidic immunoassay chip of claim 7, wherein the autoantibody target detection substance is selected from dsDNA, nucleosome, Ku antigen, histone, ssDNA, SM antigen, nRNP, RIB-P, AMA-M2, Jo-1 antigen, SSA, or SSB;
the allergen is selected from pollen, mite, mold, cat hair, dog epithelium, cockroach, house dust, or grass allergens.

9. The microfluidic immunoassay chip of claim 1, wherein the washing cell (40) is at least three washing cells (40) each being in communication with the reaction and detection cell (30).

10. The microfluidic immunoassay chip of claim 1, wherein the detection unit (100) further comprises an excess sample cell (92) being in communication with the loading cell (10), and a distance between the excess sample cell (92) and the rotation center (O) is equal to a distance between the loading cell (10) and the rotation center (O).

11. The microfluidic immunoassay chip of claim 1, wherein the detection unit (100) further comprises a venting hole (93) and a gas channel (94), the venting hole (93) is closer to the rotation center (O) than the washing cell (40), the enzyme storing cell (50), the substrate cell (60), and the termination cell (70); the washing cell (40), the enzyme storing cell (50), the substrate cell (60), and the termination cell (70) each are in communication with the venting hole (93) through the gas channel (94).

12. The microfluidic immunoassay chip of claim 11, wherein a width of the gas channel (94) is 50 µm to 100 µm, and a height of the gas channel (94) is 50 µm to 100 µm.

13. The microfluidic immunoassay chip of claim 11, further comprising a bar code (95) disposed on the detection unit (100).

14. A microfluidic line immunoassay method, comprising:
providing the microfluidic immunoassay chip of any one of claims 1 to 13;
loading a sample liquid into the loading cell (10) through the loading hole (11);
centrifuging the microfluidic immunoassay chip, releasing the sample liquid from the loading cell (10) into the reaction and detection cell (30), and incubating the sample liquid with the detection membrane strip (31);
releasing the buffer solution from the washing cell (40) into the reaction and detection cell (30) to perform a first washing;
releasing the enzyme labeled antibody from the enzyme storing cell (50) into the reaction and detection cell (30), and incubating the enzyme labeled antibody with the detection membrane strip (31);
releasing the buffer solution from the washing cell (40) into the reaction and detection cell (30) to perform a second washing;
releasing the reaction substrate from the substrate cell (60) into the reaction and detection cell (30), and incubating the reaction substrate with the detection membrane strip (31);
releasing the buffer solution from the washing cell (40) into the reaction and detection cell (30) to perform a third washing;
releasing the reaction terminating liquid from the termination cell (70) into the reaction and detection cell (30) to terminate the reaction;
detecting a reaction result from the reaction and detection cell (30).

## Patentansprüche

1. Mikrofluidik-Immunoassay-Chip, der mindestens eine Detektionseinheit (100) aufweist und einen Drehpunkt (O) aufweist, um den herum der Mikrofluidik-Immunoassay-Chip konfiguriert ist, um einen Zentrifugalbetrieb zu ermöglichen, wobei die Detektionseinheit (100) aufweist:
eine mit einer Einfüllöffnung (11) versehene Beladungszelle (10);
eine Reaktions- und Detektionszelle (30), die mit der Beladungszelle (10) in Verbindung steht;
eine Waschzelle (40), die konfiguriert ist, um mit einer Pufferlösung aufgefüllt zu werden, wobei die Waschzelle (40) mit der Reaktions- und Detektionszelle (30) in Verbindung steht;
eine Enzymspeicherzelle (50), die konfiguriert ist, dass sie mit einem enzymmarkierten Antikörper aufgefüllt wird, wobei die Enzymspeicherzelle (50) mit der Reaktions- und Detektionszelle (30) in Verbindung steht;
eine Substratzelle (60), die konfiguriert ist, dass sie mit einem Reaktionssubstrat für den enzymmarkierten Antikörper aufgefüllt wird, wobei die Substratzelle (60) mit der Reaktions- und Detektionszelle (30) in Verbindung steht;
eine Terminierungszelle (70), die so konfiguriert ist, dass sie mit einer Reaktionsterminierungsflüssigkeit aufgefüllt wird, wobei die Terminierungszelle (70) mit der Reaktions- und Detektionszelle (30) in Verbindung steht; und
eine Abfallflüssigkeitszelle (80), die so konfiguriert ist, dass sie eine Abfallflüssigkeit aufnimmt, wobei die Abfallflüssigkeitszelle (80) mit der Reaktions- und Detektionszelle (30) in Verbindung steht;
wobei die Reaktions- und Detektionszelle (30) ferner vom Drehpunkt (O) entfernt ist als die Beladungszelle (10), die Waschzelle (40), die Enzymspeicherzelle (50), die Substratzelle (60) und die Terminierungszelle (70) und näher am Drehpunkt (O) liegt als die Abfallflüssigkeitszelle (80);
**dadurch gekennzeichnet, dass** die Reaktions- und Detektionszelle (30) mit einer Vielzahl von Detektionsmembranstreifen (31) versehen ist, die mit einem Fängerantigen oder einem Fängerantikörper beschichtet sind, wobei die Reaktions- und Detektionszelle (30) eine Vielzahl von voneinander beabstandeten Detektionsschlitzen aufweist und in jeden der Vielzahl von Detektionsschlitzen entsprechend ein Detektionsmembranstreifen (31) eingebettet ist.

2. Der mikrofluidische Immunoassay-Chip nach Anspruch 1, der ferner ein Ventil (101) aufweist, das zwischen der Reaktions- und Detektionszelle (30) und jeweils der Beladungszelle (10), der Waschzelle (40), der Enzymspeicherzelle (50), der Substratzelle (60) und der Terminierungszelle (70) angeordnet ist.

3. Mikrofluidik-Immunoassay-Chip nach Anspruch 1, wobei ein Höhenunterschied zwischen einer Oberseite des Detektionsmembranstreifens (31) und einem oberen Rand einer Seitenwand jedes der mehreren Detektionsschlitze 20 µm bis 200 µm beträgt.

4. Mikrofluidik-Immunoassay-Chip nach Anspruch 1, wobei die Detektionseinheit (100) ferner eine Verdünnungszelle aufweist, die so konfiguriert ist, dass sie mit einem Verdünnungsmittel aufgefüllt ist; die Verdünnungszelle ist weiter vom Drehpunkt (O) entfernt als die Beladungszelle (10) und näher am Drehpunkt (O) als die Reaktions- und Detektionszelle (30); die Verdünnungszelle über einen Siphongang (21) mit der Beladungszelle (10) in Verbindung steht; und die Reaktions- und Detektionszelle (30) über die Verdünnungszelle mit der Beladungszelle (10) in Verbindung steht.

5. Der mikrofluidische Immunoassay-Chip nach Anspruch 4, wobei sich der Siphongang (21), der an einem Ende mit der Beladungszelle (10) in Verbindung steht, in Richtung des Drehzentrums (O) erstreckt, an einer Stelle abbiegt, die näher am Drehzentrum (O) liegt als die Beladungszelle (10), und sich in einer vom Drehzentrum (O) wegführenden Richtung erstreckt, wodurch er mit der Verdünnungszelle in Verbindung steht.

6. Der mikrofluidische Immunoassay-Chip nach Anspruch 1, wobei die Detektionseinheit (100) ferner eine mit der Beladungszelle (10) in Verbindung stehende Fällungszelle (91) aufweist und die Fällungszelle (91) weiter vom Drehpunkt (O) entfernt ist als die Beladungszelle (10).

7. Mikrofluidik-Immunoassay-Chip nach Anspruch 1, wobei das Einfangantigen eine Substanz zum Nachweis von Autoantikörper-Zielantigenen oder ein Allergen ist.

8. Mikrofluidischer Immunoassay-Chip nach Anspruch 7, wobei die Substanz zum Detektieren von Autoantikörper-Targets ausgewählt ist aus dsDNA, Nukleosom, Ku-Antigen, Histon, ssDNA, SM-Antigen, nRNP, RIB-P, AMA-M2, Jo-1-Antigen, SSA oder SSB;
das Allergen aus Pollen, Milben, Schimmelpilzen, Katzenhaaren, Hundeepithel, Kakerlaken, Hausstaub oder Grasalergenen ausgewählt ist.

9. Der mikrofluidische Immunoassay-Chip nach Anspruch 1, wobei die Waschzelle (40) aus mindestens drei Waschzellen (40) besteht, die jeweils mit der Reaktions- und Detektionszelle (30) in Verbindung stehen.

10. Mikrofluidik-Immunoassay-Chip nach Anspruch 1, wobei die Detektionseinheit (100) ferner eine Überschussprobenzelle (92), die mit der Beladungszelle (10) in Verbindung steht, aufweist, und wobei ein Abstand zwischen der Überschussprobenzelle (92) und dem Drehpunkt (O) gleich einem Abstand zwischen der Beladungszelle (10) und dem Drehpunkt (O) ist.

11. Mikrofluidik-Immunoassay-Chip, der nach Anspruch 1 beansprucht wird, wobei die Detektionseinheit (100) ferner eine Entlüftungsöffnung (93) und einen Gaskanal (94) aufweist, wobei sich die Entlüftungsöffnung (93) näher am Drehpunkt (O) befindet als die Waschzelle (40), die Enzymspeicherzelle (50), die Substratzelle (60) und die Terminierungszelle (70); die Waschzelle (40), die Enzymspeicherzelle (50), die Substratzelle (60) und die Terminierungszelle (70) jeweils über den Gaskanal (94) mit der Entlüftungsöffnung (93) in Verbindung stehen.

12. Mikrofluidik-Immunoassay-Chip nach Anspruch 11, wobei die Breite des Gaskanals (94) 50 µm bis 100 µm beträgt und die Höhe des Gaskanals (94) 50 µm bis 100 µm beträgt.

13. Mikrofluidischer Immunoassay-Chip nach Anspruch 11, der ferner einen auf der Detektionseinheit (100) angeordneten Barcode (95) aufweist.

14. Mikrofluidisches Line-Immunoassay-Verfahren, aufweisend:
Bereitstellen des mikrofluidischen Immunoassay-Chips nach einem der Ansprüche 1 bis 13;
das Einfüllen einer Probenflüssigkeit in die Beladungszelle (10) durch die Einfüllöffnung (11);
das Zentrifugieren des mikrofluidischen Immunoassay-Chips, das Freisetzen der Probenflüssigkeit aus der Beladungszelle (10) in die Reaktions- und Detektionszelle (30) sowie das Inkubieren der Probenflüssigkeit mit dem Detektionsmembranstreifen (31);
Freisetzen der Pufferlösung aus der Waschzelle (40) in die Reaktions- und Detektionszelle (30), um eine erste Waschung durchzuführen;
Freisetzen des enzymmarkierten Antikörpers aus der Enzymspeicherzelle (50) in die Reaktions- und Detektionszelle (30) und Inkubieren des enzymmarkierten Antikörpers mit dem Detektionsmembranstreifen (31);
Freisetzen der Pufferlösung aus der Waschzelle (40) in die Reaktions- und Detektionszelle (30), um eine zweite Waschung durchzuführen;
Freisetzen des Reaktionssubstrats aus der Substratzelle (60) in die Reaktions- und Detektionszelle (30) und Inkubieren des Reaktionssubstrats mit dem Detektionsmembranstreifen (31);
Freisetzen der Pufferlösung aus der Waschzelle (40) in die Reaktions- und Detektionszelle (30), um eine dritte Waschung durchzuführen;
Freisetzen der Reaktionsabbruchflüssigkeit aus der Abbruchzelle (70) in die Reaktions- und Detektionszelle (30), um die Reaktion abzubrechen;
Detektion eines Reaktionsergebnisses aus der Reaktions- und Detektionszelle (30).

## Revendications

1. Puce de dosage immunologique microfluidique, comprenant au moins une unité de détection (100) et ayant un centre de rotation (O) autour duquel la puce de dosage immunologique microfluidique est configurée pour assurer un fonctionnement de centrifugation, dans laquelle l'unité de détection (100) comprend :
une cellule de chargement (10) munie d'un trou de chargement (11) ;
une cellule de réaction et de détection (30) en communication avec la cellule de chargement (10) ;
une cellule de lavage (40) configurée pour être préremplie d'une solution tampon, la cellule de lavage (40) étant en communication avec la cellule de réaction et de détection (30) ;
une cellule de stockage d'enzyme (50) configurée pour être préremplie d'un anticorps marqué par une enzyme, la cellule de stockage d'enzyme (50) étant en communication avec la cellule de réaction et de détection (30) ;
une cellule de substrat (60) configurée pour être préremplie d'un substrat de réaction pour l'anticorps marqué par une enzyme, la cellule de substrat (60) étant en communication avec la cellule de réaction et de détection (30) ;
une cellule de terminaison (70) configurée pour être préremplie d'un liquide d'arrêt de réaction, la cellule de terminaison (70) étant en communication avec la cellule de réaction et de détection (30) ; et
une cellule de liquide usé (80) configurée pour recevoir un liquide usé, la cellule de liquide usé (80) étant en communication avec la cellule de réaction et de détection (30) ;
dans laquelle la cellule de réaction et de détection (30) est plus éloignée du centre de rotation (O) que la cellule de chargement (10), la cellule de lavage (40), la cellule de stockage d'enzyme (50), la cellule de substrat (60) et la cellule de terminaison (70), et est plus proche du centre de rotation (O) que la cellule de liquide usé (80) ;
**caractérisée en ce que** la cellule de réaction et de détection (30) est munie d'une pluralité de bandes de membrane de détection (31) revêtues d'un antigène de capture ou d'un anticorps de capture, la cellule de réaction et de détection (30) comprend une pluralité de fentes de détection espacées les unes des autres, et une bande de membrane de détection (31) est encastrée de manière correspondante dans chacune de la pluralité de fentes de détection.

2. Puce de dosage immunologique microfluidique selon la revendication 1, comprenant en outre une valve (101) disposée entre la cellule de réaction et de détection (30) et chacune de la cellule de chargement (10), la cellule de lavage (40), la cellule de stockage d'enzyme (50), la cellule de substrat (60) et la cellule de terminaison (70).

3. Puce de dosage immunologique microfluidique selon la revendication 1, dans laquelle une différence de hauteur entre une surface supérieure de la bande de membrane de détection (31) et un bord supérieur d'une paroi latérale de chacune de la pluralité de fentes de détection est de 20 µm à 200 µm.

4. Puce de dosage immunologique microfluidique selon la revendication 1, dans laquelle l'unité de détection (100) comprend en outre une cellule de dilution configurée pour être préremplie d'un diluant ; la cellule de dilution est plus éloignée du centre de rotation (O) que la cellule de chargement (10) et plus proche du centre de rotation (O) que la cellule de réaction et de détection (30) ; la cellule de dilution est en communication avec la cellule de chargement (10) par l'intermédiaire d'un canal siphoïde (21) ; et la cellule de réaction et de détection (30) est en communication avec la cellule de chargement (10) par l'intermédiaire de la cellule de dilution.

5. Puce de dosage immunologique microfluidique selon la revendication 4, dans laquelle le canal siphoïde (21), depuis l'une de ses extrémités en communication avec la cellule de chargement (10), s'étend dans un sens vers le centre de rotation (O), se courbe au niveau d'une position plus proche du centre de rotation (O) que la cellule de chargement (10), et s'étend dans un sens à l'opposé du centre de rotation (O), pour être ainsi en communication avec la cellule de dilution.

6. Puce de dosage immunologique microfluidique selon la revendication 1, dans laquelle l'unité de détection (100) comprend en outre une cellule de précipitation (91) en communication avec la cellule de chargement (10), et la cellule de précipitation (91) est plus éloignée du centre de rotation (O) que la cellule de chargement (10).

7. Puce de dosage immunologique microfluidique selon la revendication 1, dans laquelle l'antigène de capture est une substance de détection cible d'auto-anticorps ou un allergène.

8. Puce de dosage immunologique microfluidique selon la revendication 7, dans laquelle la substance de détection de cible d'auto-anticorps est choisie parmi l'ADN double brin (dsDNA), le nucléosome, l'antigène Ku, l'histone, l'ADN simple brin (ssDNA), l'antigène SM, le nRNP, le RIB-P, l'AMA-M2, l'antigène Jo-1, le SSA ou le SSB ;
l'allergène est choisi parmi le pollen, les acariens, les moisissures, les poils de chat, l'épithélium de chien, les blattes, la poussière domestique ou les allergènes de graminées.

9. Puce de dosage immunologique microfluidique selon la revendication 1, dans laquelle la cellule de lavage (40) est constituée d'au moins trois cellules de lavage (40) chacune en communication avec la cellule de réaction et de détection (30).

10. Puce de dosage immunologique microfluidique selon la revendication 1, dans laquelle l'unité de détection (100) comprend en outre une cellule d'échantillon excédentaire (92) en communication avec la cellule de chargement (10), et une distance entre la cellule d'échantillon excédentaire (92) et le centre de rotation (O) est égale à une distance entre la cellule de chargement (10) et le centre de rotation (O).

11. Puce de dosage immunologique microfluidique selon la revendication 1, dans laquelle l'unité de détection (100) comprend en outre un trou d'évent (93) et un canal de gaz (94), le trou d'évent (93) étant plus proche du centre de rotation (O) que la cellule de lavage (40), la cellule de stockage d'enzyme (50), la cellule de substrat (60) et la cellule de terminaison (70) ; la cellule de lavage (40), la cellule de stockage d'enzyme (50), la cellule de substrat (60) et la cellule de terminaison (70) sont chacune en communication avec le trou d'évent (93) par l'intermédiaire du canal de gaz (94).

12. Puce de dosage immunologique microfluidique selon la revendication 11, dans laquelle une largeur du canal de gaz (94) est de 50 µm à 100 µm, et une hauteur du canal de gaz (94) est de 50 µm à 100 µm.

13. Puce de dosage immunologique microfluidique selon la revendication 11, comprenant en outre un code-barres (95) disposé sur l'unité de détection (100).

14. Procédé de dosage immunologique linéaire microfluidique, comprenant :
la fourniture de la puce de dosage immunologique microfluidique selon l'une quelconque des revendications 1 à 13 ;
le chargement d'un liquide échantillon dans la cellule de chargement (10) à travers le trou de chargement (11) ;
la centrifugation de la puce de dosage immunologique microfluidique, la libération du liquide échantillon depuis la cellule de chargement (10) jusque dans la cellule de réaction et de détection (30), et l'incubation du liquide échantillon avec la bande de membrane de détection (31) ;
la libération de la solution tampon depuis la cellule de lavage (40) jusque dans la cellule de réaction et de détection (30) pour effectuer un premier lavage ;
la libération de l'anticorps marqué par une enzyme depuis la cellule de stockage d'enzyme (50) jusque dans la cellule de réaction et de détection (30), et l'incubation de l'anticorps marqué par une enzyme avec la bande de membrane de détection (31) ;
la libération de la solution tampon depuis la cellule de lavage (40) jusque dans la cellule de réaction et de détection (30) pour effectuer un deuxième lavage ;
la libération du substrat de réaction depuis la cellule de substrat (60) jusque dans la cellule de réaction et de détection (30), et l'incubation du substrat de réaction avec la bande de membrane de détection (31) ;
la libération de la solution tampon depuis la cellule de lavage (40) jusque dans la cellule de réaction et de détection (30) pour effectuer un troisième lavage ;
la libération du liquide d'arrêt de réaction depuis la cellule de terminaison (70) jusque dans la cellule de réaction et de détection (30) pour arrêter la réaction ;
la détection d'un résultat de réaction en provenance de la cellule de réaction et de détection (30).
